(19) Europäisches Patentamt
European Patent Office
Office européen des brevets

(11) Publication number: 0 246 786
A1

(12) # EUROPEAN PATENT APPLICATION

(21) Application number: 87304115.6

(22) Date of filing: 08.05.87

(51) Int. Cl.$^3$: C 07 D 417/14
C 07 D 401/14
//C07D205/08, A61K31/64

(30) Priority: 23.05.86 US 866792

(43) Date of publication of application:
25.11.87 Bulletin 87/48

(84) Designated Contracting States:
AT ES GR

(71) Applicant: THE UPJOHN COMPANY
301 Henrietta Street
Kalamazoo, Michigan 49001(US)

(72) Inventor: Magerlein, Barney
c/o The Upjohn Company 301 Hentietta Street
Kalamazoo Michigan 49001(US)

(72) Inventor: Kim, Kyoung S.
c/o The Upjohn Company 301 Hentietta Street
Kalamazoo Michigan 49001(US)

(74) Representative: Perry, Robert Edward et al,
GILL JENNINGS & EVERY 53-64 Chancery Lane
London WC2A 1HN(GB)

(54) **N-1 subsituted sulfonylaminocarbonyl, C-4 substituted monobactams.**

(57) This invention presents novel 2-azetidinone compounds as shown in formula I, which are useful as antibacterial agents to eradicate or control susceptible microbes. These compounds have a (5-hydroxy-4-pyridone-2-yl)carbonylamino-1-(2-imidazolidone-3-yl)sulfonylaminocarbonyl group at the N–1 position, and a carbonate-type substituent or an amino acid derivative at the C–4 position via an ester linkage. Intermediates and processes for making these compounds are also disclosed.

EP 0 246 786 A1

# N-1 SUBSTITUTED SULFONYLAMINOCARBONYL, C-4 SUBSTITUTED MONOBACTAMS

## FIELD OF THE INVENTION

This invention concerns novel 2-azetidinone compounds having antimicrobial activity.

## INFORMATION DISCLOSURE

Analogs of 2-azetidinone derivatives having antimicrobial and β-lactamase inhibitory activity are known in the art. Takeda European patent applications 0-053-815 and 0-053-816, Squibb European patent application 0-076-758, and Hoffman-LaRoche European patent application 0-096-297. Each of these applications discloses β-lactams with various substituents at the C-4 position of the ring.

The Takeda patent application EP 0-053-816 discloses 2-azetidinone compounds and generally describes the C-4 position as substituted by an organic residue. However, none of the more detailed descriptions suggests the specific substituents disclosed herein, nor does the disclosure suggest or teach how to make the specific compounds of this application.

Abstracts, 25th ICAAC, Minneapolis, MN, October 1985, Squibb Institute for Medical Research, disclose compound SQ 83,360, having a substituted-sulfonylaminocarbonyl group at the N-1 position and a 2-(2-amino-4-thiazolyl)-2-(1-carboxy-1-methylethoxy)-iminoacetamido group at the C-3 position, as an antibacterial agent.

## SUMMARY OF THE INVENTION

The present invention relates to novel 2-azetidinone analogs and to their uses as microbial growth inhibitors. Included are both the racemic mixtures and optical isomers of these compounds. Also disclosed are intermediates and processes useful for preparing these compounds.

As illustrated in the Formula Chart, the present invention provides (refer to Formulas, Chart A and claim 1 below):

A. A compound of the formula I

wherein the $R_{20}NH$- and $-CH_2OR_{10}$ groups are either cis or trans to each other;

wherein n is 1 or 2;

wherein $R_{10}$ is

a) $-C(=O)CH_2NHR_4$, or

b) $-C(=O)OR_{20}$;

provided that n is 2 only when $R_{10}$ is $-C(=O)CH_2NHR_4$;

wherein $R_4$ is

a) hydrogen,

b) $-C(=O)H$, or

c) t-butyloxycarbonyl;

wherein $R_{30}$ is

a) $-(C_1-C_8)$alkyl, or

b) $-(CH_2)_2X$ wherein X is $-NH_2$, -NH-(t-butyloxycarbonyl), $-NHCH_3$, $-N(CH_3)$-(t-butyloxycarbonyl), $-OC(=O)NH_2$, -Cl, $-OCH_3$ or $-NH-C(=O)H$;

wherein $R_{20}$ is

a) hydrogen, or

b) a Z-oximino-acyl moiety of formula II

wherein $R_{40}$ is

a) $-CH_3$,

b) $-CH_2COOR_{41}$,

c) $-C(CH_3)_2COOR_{41}$, or

d) $-CH(CH_3)COOR_{41}$;

wherein $R_{41}$ is

a) hydrogen,

b) -t-butyl,

c) diphenylmethyl, or

d) benzyl;

wherein $R_{50}$ is

a) hydrogen,

b) t-butyloxycarbonyl,

c) benzyloxycarbonyl, or

d) triphenylmethyl;

wherein $R_{60}$ is an imidazolidinone moiety of formula III or a pharmaceutically acceptable salt thereof.

B. Novel chemical intermediates:

(1) compounds of the formula A-7 wherein n is 1 or 2, $R_4$ is $-C(=O)H$ or t-butyloxycarbonyl (BOC), $R_{20}$ is a Z-oximino-acyl moiety of the formula II wherein $R_{40}$ is as defined above, $R_{41}$ is t-butyl, diphenylmethyl or benzyl, $R_{50}$ is t-butyloxycarbonyl (BOC), benzyloxycarbonyl (Cbz) or triphenylmethyl (trityl), and $R_{60}$ is a moiety of the formula III; and compounds of the formula A-6 wherein n is 1 or 2, $R_{14}$ is $-C(=O)H$ or t-butyloxycarbonyl (BOC), $R_{21}$ is a Z-oximino-acyl

moiety of the formula II wherein $R_{40}$ is as defined above, $R_{41}$ is t-butyl, diphenylmethyl or benzyl, $R_{50}$ is t-butyloxycarbonyl (BOC), benzyloxycarbonyl (Cbz), or triphenylmethyl (Trityl) and $R_{60}$ is a moiety of the formula III.

(2) compounds of the formula A-4', A-5' and A-6' wherein $R_{31}$ is $-(C_1-C_8)$alkyl or $-(CH_2)_2X$ wherein X is -NH-(t-butyloxycarbonyl) [-NHBOC], $-N(CH_3)$-(t-butyloxycarbonyl) [$-N(CH_3)BOC$], $-OC(=O)NH_2$, -Cl, $-O-CH_3$ or -NHC(=O)H, and $R_{60}$ is a moiety of the formula III;

(3) compounds of the formula A-7' wherein $R_{20}$ is Z-oximino-acyl a moiety of the formula II wherein $R_{40}$ is defined as above, $R_{41}$ is t-butyl, diphenylmethyl or benzyl, $R_{50}$ is t-butyloxycarbonyl (BOC), benzyloxycarbonyl (Cbz) or triphenylmethyl (trityl), $R_{30}$ is $-(C_1-C_8)$-alkyl or $-(CH_2)_2X$ wherein X is -NH-(t-butyloxycarbonyl) [-NHBOC], $-N(CH_3)$-(t-butyloxycarbonyl) [$-N(CH_3)BOC$], $-OC(=O)NH_2$, -Cl, $-OCH_3$ or -NH-C(=O)H, and $R_{60}$ is a moiety of the formula III; and acid addition, alkalai metal and internal salts thereof. These novel intermediates are generally less important as antibacterial agents and more important as chemical intermediates to antibacterial compounds of this invention.

Unless otherwise indicated, the following definitions are used herein:

The carbon atom content of various hydrocarbon-containing moieties as indicated by a prefix designating the minimum and maximum number of carbon atoms in the moiety, i.e., the prefix $(C_i-C_j)$ indicates a moiety of the integer "i" to the integer "j" carbon atoms, inclusive. Thus, for example, $(C_1-C_3)$ alkyl refers to alkyl of one to three carbon atoms, inclusive, or methyl, ethyl, propyl, and isopropyl.

Examples of alkyl of one to eight carbon atoms, inclusive, are methyl, ethyl, propyl, butyl, pentyl, hexyl, heptyl, and octyl and isomeric forms thereof.

The oximino compounds of this invention are in the Z-isomer form. This Z-isomer form is illustrated in the molecular fragment of the formula II: i.e., the $R_{40}O$- is on the same "side" of the carbon-nitrogen double bond as the -C(=O)- (higher Cahn-Ingold-Prelog priority) and thus this isomer is termed the Z-isomer (Z-of the German "zusammen", "together").

The term "pharmaceutically acceptable salts" includes those compounds created by salt formation with the sulfonylaminocarbonyl group at the N-1 position as well as those formed with the amino, amide or

carboxyl moieties of substituents on positions C-3 and C-4. These include salts with acids, and with alkali methal bases. Internal salts, zwitterionic compounds are also part of this invention. Such salts are made by known methods.

Acid salts are formed by reacting the compounds described herein with the appropriate acid in a suitable solvent. Suitable acids for this purpose include hydrochloric, sulfuric, phosphoric, hydrobromic, hydroiodic, acetic, lactic, citric, succinic, benzoic, salicylic, pamoic, cyclohexansulfamic, methanesulfonic, naphthalenesulfonic, p-toluenesulfonic, maleic, fumaric, oxalic and the like.

Metal salts are formed by suspending the compounds in water or other suitable solvent and adding a dilute metal base such as sodium or potassium bicarbonate until the pH is between 6 and 7. Metal salts include sodium and potassium salts.

It will be recognized by those skilled in the art that carbon atoms $C_3$ and $C_4$ of the 4-membered ring of compounds of formula I of this invention are asymmetrically substituted and can independently possess the R or S-configuration. There are thus 4 stereoisomers which comprise two pairs of enantiomers; each enantiomeric pair is termed a racemate. Of the two racemates, one will have the $R_{20}NH$- and $-CH_2OR_{10}$ groups cis to each other (on the same side of the ring) and the other pair will have the $R_{20}NH$- and $-CH_2OR_{10}$ groups trans to each other (or opposite sides of the ring). The cis compounds of this invention are preferred, and the cis compounds having the S-configuration at each of $C_3$ and $C_4$ of the ring are more preferred. This invention includes within its scope all enantiomeric or diastereomeric forms of formula I compounds either in pure form or as mixtures of enantiomers or diastereomers.

With the possible exception of the chemical intermediates of this invention described above, the compounds of the formula I of this invention and their respective pharmaceutically acceptable salts have valuable antibiotic activity against a variety of gram-negative bacteria including Escherichia coli, Klebsiella pneumoniae, and Pseudomonas aeruginosa. The compounds are useful for treating bacterial infections in animals, including and most preferably humans.

Compounds of the invention are tested for in vitro antimicrobial activity using standard testing procedures such as the determination of minimum inhibitory concentration (MIC) by agar dilution as described in

"Methods for Dilution Antimicrobial Susceptibility Tests for Bacteria That Grow Aerobically" (MF-T) Published January 1983 by the National Committee for Clinical Laboratory Standards, 771 East Lancaster Avenue, Villanova, PA 19084. Briefly, MIC values are determined in unsupplemented Mueller Hinton Agar (MHA). The compounds tested are diluted serially into molten MHA at 47°C. The agar is poured into petri dishes and allowed to harden. The various bacteria used for testing are grown overnight on MHA at 35°C and transferred to Tryptiease Soy Broth (TSB) until a turbidity of 0.5 McFarland standard is obtained. The bacteria are diluted one to twenty in TSB and inoculated on the plates (1 $\mu$l using a Steers replicator). The plates are incubated at 35°C for 20 hours and the MIC is read to be the lowest concentration of drug that completely inhibits visible growth of the bacterium. The MIC test results of a compound of this invention (Compound A) is given in Table I.

The process for making compounds of Formula I is illustrated in Charts A, B and C. The requirements for protecting groups in the processes of Charts A-C are generally well recognized by one skilled in the art of organic chemical synthesis. The use of suitable protecting groups in the processes of Charts A-C is understood. It is recognized that conditions for introduction and removal of protecting groups should not undesirably alter any other groups in the molecule.

Examples of suitable nitrogen protecting groups are:

(1) benzyl ($C_6H_5$-$CH_2$-);

(2) triphenylmethyl(trityl, $C_6H_5)_3C$);

(3) trialkylsilyl, for example, trimethylsilyl ($(CH_3)_3Si$-) or tertiary butyldimethylsilyl ($(CH_3)_3Si(CH_3)_2$-) and the like,

(4) tert-butoxycarbonyl (t-BOC or BOC),

(5) benzyloxycarbonyl (Cbz),

(6) trifluoroalkanoyl, e.g., trifluoroacetyl, trifluoropropionyl, and

(7) diphenyl(methyl)silyl, and the like.

Introduction and removal of such nitrogen protecting groups are well known in the art of organic chemistry: See, for example, (1) J.F.W. McOmie, Advances in Organic Chemistry, Vol. 3, pages 191-281 (1963); (2) R.A. Boissonas, Advances in Organic Chemistry, Vol. 3, pages 159-190 (1963); (3) "Protective Groups in Organic Chemistry", J.F.W. McOmie, Ed., Plenum Press, New York, 1973, pg 74, and (4) "Protective

Groups in Organic Synthesis", Theodora W. Greene, John Wiley and Sons, New York, 1981.

Under certain circumstances it may be necessary to protect two (or more) different nitrogens with different protecting groups os that one such protecting group can be selectively removed while leaving the second protecting group in place. For example, the Cbz and BOC protecting groups can be used in this way, the Cbz group being removable in the presence of the BOC group and vice versa.

Chart A

In Chart A, the starting compound cis-(±)-4-(methoxycarbonyl)-3-[[(benzyloxy)carbonyl]amino]-2-azetidinone (A-1) is known. J. Org.-Chem., 47:2765-2767 (1982). The trans compound A-1 is known or is made by known methods. Thus compound A-1 is either cis or trans with respect to the substituents on C-3 and C-4. It is recognized that alternative alkoxycarbonyls of from 1-5 carbon atoms, inclusive or aralkoxycarbonyls of from 5-14 carbon atoms, inclusive, either unsubstituted or substituted by 1-4 groups of halogen, nitro, or $(C_1-C_4)$ alkyl could be used in place of the benzyloxycarbonyl group of compound A-1. See also, W. F. Huffman et al., J. Am. Chem. Soc., 99:2352 (1977); D. B. Bryan et al., J. Am. Chem. Soc., 99:2353 (1977).

The C-4-carbomethoxy group of compound (A-1) is reduced to the C-4-hydroxymethyl group of compound (A-2) by use of metal hydride reducing reagents, such as sodium borohydride or zinc borohydride, in ether solvents, such as diethyl ether or tetrahydrofuran, at a temperature range of 0° to 80°C. The product is obtained after a normal aqueous work-up procedure followed by column chromatography on silica gel.

The sequence A-2 → A-7 of Chart A is used to produce the C-4-glycinoyloxymethyl-substituted compounds (A-7) of the present invention. The benzyloxycarbonyl group of compound (A-2) is removed catalytic hydrogenolysis using palladium metal supported on carbon or palladium metal itself under a hydrogen gas atmosphere in various kinds of organic solvents, such as alcoholic solvents, ether solvents or ethyl acetate solvent, at ambient temperature. The compound (A-3) is obtained by filtration of the solid catalyst followed by concentration under reduced pressure.

The C-3-amino group of compound (A-3) is acylated with a suitably protected known acid, $R_{21}$-OH wherein $R_{21}$ is a Z-oximino-acyl moiety of

formula II wherein $R_{40}$ is defined as above, $R_{41}$ is t-butyl, diphenylmethyl or benzyl, and $R_{50}$ is BOC, Cbz or trityl, to produce compound (A-4). This conversion may be carried out by any of a number of amide or peptide forming reaction sequences such as described in Methoden der Organischem Chemie, Vierte Auflage, Band XV/2, E. Wunch ed., Georg Thieme Verlag, Stuttgart, p.1. A preferred acylation process is the use of approximately equimolar quantities of a desired acid, 1-hydroxy-1-benzotriazole (HOBT), and a carbodiimide, such as dicyclohexylcarbodiimide (DCC). The choice of solvents is methylene dichloride, dimethylformamide or a combination of both solvents and the reaction is carried out in general at the temperature range of 0°C to ambient temperature. The desired compound (A-4) is obtained after filtration of precipitated dicyclohexylurea and removal of HOBT by treating with aqueous sodium bicarbonate solution and then column chromatography on silica gel. Also, the compound (A-4) can be made by methods known in the art, i.e., J. Am. Chem. Soc., 95:2401-2404 (1973).

The hydroxymethyl group of compound (A-4) is reacted with HOC(=O)-$CH_2NHR_{14}$ wherein $R_{14}$ is -C(=O)H or BOC to produce compound A-5 using the DCC/HOBT method in the presence of a catalytic amount of 4-dimethylamino-pyridine in solvents, such as methylene dichloride, dimethylformamide or a combination of both solvents, at a temperature range of 0°C to ambient temperature. When the reaction is complete, dicyclohexylurea and HOBT are removed by filtration of the precipitated solid and washing with aqueous sodium bicarbonate, respectively. The compound A-5 is purified by column chromatography on silica gel.

The compound (A-5) is treated with approximately 1.2 to 1.6 equivalents of chlorosulfonyl isocyanate at -20° to 0°C in organic solvents, such as methylene dichloride, acetonitrile or a combination of both solvents to produce compounds (A-6) with n=1 and 2, which are used directly in the next step. Generally the greater the amount of chlorosulfonyl isocyanate used, the greater the amount of compound (A-6) with n=2 produced.

The imidazolidinone of Formula IV is silylated by reaction with N-methyl-N-(trimethylsilyl)-trifluoroacetamide or bis-(trimethylsilyl)-trifluoroacetamide in organic solvents, such as acetonitrile, methylene dichloride or tetrahydrofuran, at ambient temperature. This silylated imidazolidinone is reacted with compound (A-6) in the presence of a tertiary amine base such as 2,6-lutidine at 0°C, and the mixture is

slowly warmed to room temperature over a period of 1 to 5 hours. The crude product is obtained after a normal aqueous work-up procedure and then deprotected by treatment with trifluoroacetic acid (or deprotected otherwise as appropriate) followed by purification, e.g., by column chromatography on Diaion HP-20 resin (Mitsubishi Chemical Co.) or XAD-resin (Rohm and Haas Co.) to produce the final N-1-sulfonyl-aminocarbonyl activated compound (A-7).

The sequence A-2 → A-7' of Chart A is used to produce the C-4 $R_{30}$-oxycarbonyloxymethyl-substituted compounds (A-7') of the present invention. The hydroxymethyl compound (A-2) is treated with a suitable protected chloroformate ester of formula $ClCOOR_{31}$ where $R_{31}$ is defined as $-C_1-C_8$)alkyl or $-(CH_2)_2X$ wherein X is -NHBOC, $-N(CH_3)BOC$, -OC(=O)-$NH_2$, Cl, $OCH_3$ or -NHC(=O)H, to give the compound (A-3'). This is the preferred route to the compound (A-3'). The reaction conditions involve the use of an inert solvent such as methylene dichloride, tetrahydrofuran, or dimethylformamide at -20°C to 30°C in the presence of a slight excess of organic base, such as pyridine, 2,4-lutidine, or triethylamine. Following extractive workups involving successive washes with acid and base, the products are isolated by chromatography or crystallization. Some chloroformate esters are commercially available and others are be prepared according to the teaching of Huntress, "Organic Chlorine Compounds," John Wiley and Sons, Inc., New York, NY, 1948; F. Stain et al., J. Am.Chem. Soc., 72, 1254 (1950), H. G. Ashburm et al., J. Am. Chem. Soc., 60, 2933 (1938). Briefly, the process described in these references is to contact an alcohol with an excess of phosgene either neat or in an organic solvent. After workup, the product is usually isolated by vacuum distillation.

An alternative process to prepare compounds (A-3') is available. This process is helpful when the desired chloroformate is unavailable. Compound (A-2) is placed in a solvent such as methylene dichloride, ethyl acetate, tetrahydrofuran, or acetonitrile containing a slight excess of an organic base, such as pyridine, triethylamine, or 2,4-lutidine, and is reacted at -20°C to 30°C with a solution of phosgene in an inert solvent, such as toluene, benzene or methylene dichloride. The intermediate chloroformate thus formed is not isolated, but is treated with a molar equivalent of the suitably protected desired alcohol ($R_{31}OH$), wherein $R_{31}$ is $(C_1-C_8)$alkyl or $(CH_2)_2X$ wherein X is-NH-BOC, $-N(CH_3)BOC$, $-OC(=O)NH_2$, Cl, $OCH_3$ or NH-C(=O)H, in the presence

of an organic base in an inert solvent at -20° C to 30° C to yield the compound (A-3'). This alternative or reversed process is known in the field of steroid chemistry, G. Schubert et al., Die Pharmazie, 35, 453 (1980).

The compound (A-3') is treated with chlorosulfonyl isocyanate at -15° to 25°C in organic solvents, such as methylene dichloride, acetonitrile or a combination of both solvents to produce compound (A-4') which is used in the next step without purification. The imidazolidinone of Formula IV is silylated by reaction with N-methyl-N-(trimethylsilyl)-trifluoroacetamide or bis-(trimethylsilyl)-trifluoroacetamide in organic solvents, such as ethyl acetate, acetonitrile, methylene dichloride or tetra-hydrofuran, at ambient temperature. This silylated imidazolidinone is reacted with compound (A-4') at 0°C, and the mixture is slowly warmed to room temperature over a period of 1 to 5 hours. The crude product is purified by conventional methods to produce the N-1-sulfonylaminocarbonyl activated compound (A-5').

The benzyloxycarbonyl group of compound (A-5') is removed by catalytic hydrogenolysis using palladium metal supported on carbon or palladium metal itself under a hydrogen gas atmosphere in various kinds of organic solvents, such as dimethylformamide, alcoholic solvents, ether solvents or ethyl acetate solvent, at ambient temperature. The compound (A-6') is obtained by filtration of the solid catalyst followed by concentration under reduced pressure.

The C-3-amino group of compound (A-6') is acylated with a suitably protected known acid $R_{21}$-OH, wherein $R_{21}$ is as defined above, followed by deprotection as appropriate to produce compound (A-7'). This conversion may be carried out by any of a number of amide or peptide forming reaction sequences as described above for the conversion of compound A-3 to A-4.

Optically active compounds of the formula I of this invention are prepared by the use in the processes of Chart A of the appropriate optically active form of compound (A-1), which is prepared by known methods. Takeda European patent application 8310461-3. The resolving agents are any of the known resolving agents such as optically active camphorsulfonic acid, bis-o-toluoyltartaric acid, tartaric acid, and diacetyl tartaric acid which are commercially available and which are commonly used for resolution of amines (bases), as for example in

Organic Synthesis, Coll. Vol. V., p. 932 (1978), resolution of R-(+) and S-(-)-α-phenylethylamine with (-)-tartaric acid.

The preferred starting compound for making optically active compounds of Formula I, cis-(±)-1-[(2',4'-dimethoxyphenyl)methyl]-4-(methoxycarbonyl)-3-phenylmethoxycarboxyamino-2-azetidinone, is known. Chem. Pharm. Bull., 32:2646-2659 (1984). The C-3 protecting group is removed by hydrogenolysis to produce the corresponding free amine. An appropriate substituted tartaric acid enantiomer is then added such as (+)-di-p-toluoyl-D-tartaric acid and reaction conditions altered to facilitate precipitation of the appropriate azetidinone diastereomeric salt. The tartaric acid is removed by treating the compound with inorganic base such as aqueous sodium bicarbonate to produce the desired resolved C-3-amino-azetidinone.

The compounds of this invention are administered to human and other animal subjects by parenteral (including intravenous and intramuscular) and rectal routes.

Various compositions of the compounds of the present invention are presented for administration to humans and animals in unit dosage forms. The term "unit dosage form", as used in this specification, refers to physically discrete units suitable as unitary dosages for human subjects and animals, each unit containing a predetermined quantity of active material calculated to produce the desired pharmaceutical effect in association with the required pharmaceutical diluent, carrier or vehicle. The specifications for the novel unit dosage forms of this invention are dictated by and directly dependent on (a) the unique characteristics of the active material and the particular effect to be achieved and (b) the limitations inherent in the art of compounding such an active material for use in humans and animals. Examples of suitable unit dosage forms in accord with this invention are ampoules, vials, suppositories, segregated multiples of any of the foregoing, and other forms as herein described.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampoule and sealing. Advantageously, adjuvants such as a local anesthetic,

preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Additionally, a rectal suppository can be employed to deliver the active compound. This dosage form is of particular interest where the mammal cannot be treated conveniently by means of other dosage forms, as in the case of young children or debilitated persons. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. These rectal suppositories can weigh from about 1 to 2.5 gm.

An effective quantity of the compound is employed in treatment. The dosage of the compound of this invention for treatment depends on many factors that are well known to those skilled in the art. They include for example, the route of administration and the potency of the particular compound. For treating bacterial infections a dosage schedule for humans having an average weight of 70 kilograms is from about 50 to about 3,000 mg of compound in a single dose, administered from 1 to 4 times per day parenterally in the compositions of this invention. Equivalent dosages are employed for other routes of administration. More specifically, the preferred single dose is from about 100 mg to 2,000 mg of compound. The rectal dose is from about 100 mg to about 4,000 mg in a single dose.

DESCRIPTION OF THE PREFERRED EMBODIMENTS

Without further elaboration, it is believed that one skilled in the art can, using the preceding description, practice the present invention to its fullest extent. The following detailed examples

describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

Preparation 1 cis-(±)-4-(Hydroxymethyl)-3-[((phenylmethoxy)-carbonyl)amino]-2-azetidinone (B-2) (A-1). Refer to Chart B.

To a stirred solution of zinc chloride (23.2 g) in anhydrous tetrahydrofuran (300 ml) at 0°C is added sodium borohydride (13.8 g) and the mixture is allowed to warm to room temperature and is stirred overnight. To the mixture is added cis-(±)-4-(methoxycarbonyl)-3-[((phenylmethoxy)carbonyl)amino]-2-azetidinone (B-1) (39.2 g) and the reaction mixture is slowly heated to 65°C and stirred at that temperature for 2 hours. The reaction mixture is cooled to 0°C and 6 N hydrochloric acid (200 ml) is added dropwise with stirring. The mixture is poured into ethyl acetate (1 l) and the organic layer is taken. The aqueous layer is saturated with sodium chloride and reextracted with ethyl acetate (200 ml). The combined organic layer is washed with water (200 ml) and with 200 ml of brine twice and dried over anhydrous sodium sulfate. The solvent is concentrated under reduced pressure to afford a yellow oil which is purified by column chromatography on silica gel (ethyl acetate as eluent) to obtain the title product (B-2) as a white solid (24.3 g).

Physical characteristics are as follows:

MP: 98-100°C.

1H NMR ($\delta$, $CD_3OD$) 7.3, 5.2, 5.1, 3.75.

IR ($cm^{-1}$, neat) 3300, 1745, 1702.

FAB-MS found: 251.1042.

Preparation 2 cis-(±)-3-[2[(2-t-Butoxycarbonylamino-4-thiazolyl]-2-[(1-t-butoxycarbonylmethoxy)imino]]acetamido-4-hydroxymethyl-2-azetidinone (B-4: $R_{40}$=1-t-butoxy-carbonylmethyl). Refer to Chart B.

To a stirred solution of the title product (B-2) of Preparation 1 (19.5 g) in methanol (150 ml) is added palladium black (7.6 g) slurried in ethanol (25 ml) and the reaction mixture is stirred under 1 atm of hydrogen gas for 24 hours. Toluene (100 ml) is added to the reaction

mixture and it is stirred for 15 minutes. The solid material is filtered and the filtrate solution is concentrated under reduced pressure to obtain 3-amino-4-hydroxymethyl-2-azetidinone (B-3) which is used directly for the next step.

The compound (B-3) obtained above is dissolved in methylene dichloride (200 ml) and dimethylformamide (500 ml) and cooled in the ice bath. To this cooled solution, 2-[(2-t-butoxycarbonylamino)-4-thiazolyl]-[(1-t-butoxycarbonylmethoxy)imino]-carboxylic acid (23.8 g) is added followed by dicyclohexylcarbodiimide (12.6 g) and 1-hydroxybenzotriazole (4.2 g). The reaction mixture is stirred for 3 hours at 0°C. The precipitated solid is filtered and the filtrate solution is partitioned between ethyl acetate (2.5 l) and water (1 l). The organic layer is taken and the aqueous layer is washed with 500 ml of ethyl acetate twice. The combined organic layer is washed with aqueous sodium bicarbonate followed by brine and dried over anhydrous sodium sulfate. It is filtered and the filtrate solution is concentrated under reduced pressure and the residual material is chromatographed on silica gel eluting with hexane:ethyl acetate/1:1 followed by ethyl acetate alone to obtain 14.1 g of the title product (B-4).

Physical characteristics are as follows:

MP: 195°C. (decomp.).

1H NMR (δ, $CDCl_3$) 8.4, 8.2, 7.35, 6.45, 5.5, 4.68, 4.2-3.7, 1.6, 1.50, 1.44.

Preparation 3 cis-(±)-3-[2[(2-t-Butoxycarbonylamino-4-thiazolyl]-2-[(1-t-butoxycarbonylmethoxy)imino]]acetamido-4-N-formyl-glycinoyloxymethyl-2-azetidinone (B-5: $R_{40}$=1-t-butoxycarbonylmethyl, $R_{14}$=-C(=O)H). Refer to Chart B.

To a mixture of the title product (B-4) of Preparation 2 (4.5 g), 1-hydroxybenzotriazole (1.216 g), dimethylaminopyridine (122 mg), N-formylglycine (1.62 g) and in the presence of a small amount of 4A molecular sieves in 60 ml of methylene dichloride and 6 ml of dimethylformamide, dicyclohexylcarbodiimide (3.25 g) is added with stirring at room temperature. The reaction is complete in 2 hours. The precipitated solid is filtered off, washed with methylene dichloride (50 ml) and the filtrate solution is stirred with aqueous sodium bicarbonate (1.89 g sodium bicarbonate in 40 ml water) at room temperature for 15 minutes. The organic layer is taken, dried over sodium sulfate and

concentrated under reduced pressure. The residue is passed through the medium pressure silica gel column eluting with 3:1 hexane/ethyl acetate followed by ethyl acetate to obtain 3.6 g of the title product (B-5).

Physical characteristics are as follows:

MP: 108-110°C.

1H NMR (δ, $CDCl_3$) 8.85, 8.55, 7.35, 7.25, 7.0, 5.35, 4.63, 4.5-4.0, 1.53, 1.45.

Preparation 4 Alternative preparation of cis-(±)-4-(Hydroxymethyl)-3-[((phenylmethoxy)carbonyl)amino]-2-azetidinone (C-1) (B-2). Refer to Chart C.

A solution of 1.63 g of sodium borohydride in 25 ml of water is added dropwise to a well stirred soution of 3.0 g of cis-(±)-4-(methoxycarbonyl)-3-[((phenylmethoxy)carbonyl)amino]-2-azetidinone (B-1) in 190 ml of tetrahydrofuran. The addition is made over a period of 10 minutes while stirring in an ice bath. The reaction is stirred for 3 hours. Methylene dichloride (250 ml) is added followed by anhydrous sodium sulfate. A clear solution is obtained by filtration. The solvent is distilled. The residue is dissolved in acetone. The solution is clarified by filtration and concentrated. A hard crystalline residue of 2.84 g of the title product (C-1) is obtained.

Physical characteristics are as follows:

TLC: One spot, slower than starting material, is observed on TLC (cyclohexane-acetone, 1:1).

$^{13}C$ NMR (δ, $CH_3OH-d_6$): 55.9, 60.2, 61.6, 67.9, 128.7-129.3, 137.6, 158, 170.7.

FAB exact mass of [M·+K]+ found: 289.0589.

Preparation 5 cis-(±)-4-[Methoxycarbonyl)oxymethyl]-3-[((phenylmethoxy)-carbonyl)amino]-2-azetidinone (C-2: $R_{31}=CH_3$). Refer to Chart C.

Methyl chloroformate (2.42 g) is added dropwise to a solution of 3.2 g of the title product (C-1) of Preparation 4 and 3.03 g of pyridine in 100 ml of methylene dichloride while stirring at 0°C. After 1 hour an additional 0.5 g of methyl chloroformate is added. The reaction mixture is stirred an additional 0.5 hour and then washed successively with dilute mineral acid (e.g., HCl or $H_2SO_4$), water and potassium bicarbonate solution. Evaporation of the solvent and trituration of the residue with ethyl acetate affords 2.48 g of the title product (C-2) as a white solid. An additional 0.75 g of the

title product is obtained by partial concentrations of the trituration solvent.

Physical characteristics are as follows:

MP: 155-158°C.

$^{13}C$ NMR (δ, $Me_2CO-d_6$): 52.1, 54.6, 60.4, 66.7, 67.4, 128.0, 128.1, 128.6, 137, 156, 167, 176.

According to the procedures of Preparation 5, the following compounds are also prepared:

cis-(±)-4-[(Formylaminoethoxycarbonyl)oxymethyl]-3-[((phenylmethoxy)carbonyl)amino]-2-azetidinone (C-2): $R_{30}$=$CH_2CH_2NHC(=O)H$;

cis-(±)-4-[(t-Butoxycarbonylaminoethoxycarbonyl)oxymethyl]-3-[((phenylmethoxy)carbonyl)amino]-2-azetidinone (C-2): $R_{30}$=$CH_2CH_2NH$-BOC(t);

cis-(±)-4-[(Aminocarbonyloxyethoxycarbonyl)oxymethyl]-3-[((phenylmethoxy)carbonyl)amino]-2-azetidinone (C-2): $R_{30}$=$CH_2CH_2OC(=O)NH_2$; and

cis-(±)-4-[(Chloroethoxycarbonyl)oxymethyl]-3-[((phenylmethoxy)-carbonyl)amino]-2-azetidinone (C-2): $R_{30}$=$CH_2CH_2Cl$.

Preparation 6 cis-(±)-1-[(Chlorosulfonyl)aminocarbonyl]-4-[(methoxycarbonyl)oxymethyl]-3-[((phenylmethoxy)-carbonyl)amino]-2-azetidinone (C-3: $R_{31}$=$CH_3$). Refer to Chart C.

Chlorosulfonyl isocyanate (175 mg) is added dropwise to a suspension of 382 mg of the title product (C-2) of Preparation 5 in 8 ml of methylene dichloride while stirring in an ice bath. The bath is removed after 20 minutes and the mixture stirred at ambient conditions. Evaporation of the solvent under vacuum leaves the title product (C-3) as a glass which is used without purification.

According to the procedure of Preparation 6, the following chlorosulfonyl compounds are also prepared:

cis-(±)-1-[(Chlorosulfonyl)aminocarbonyl]-4-[(formylaminoethoxycarbonyl)-oxymethyl]-3-[((phenylmethoxy)carbonyl)amino]-2-azetidinone (C-3): $R_{30}$=$CH_2CH_2NHC(=O)H$;

cis-(±)-1-[(Chlorosulfonyl)aminocarbonyl]-4-[(t-butoxycarbonylaminoethoxycarbonyl)-oxymethyl]-3-[((phenylmethoxy)carbonyl)amino]-2-azetidinone (C-3): $R_{30}$=$CH_2CH_2NBOC(t)$;

cis-(±)-1-[(Chlorosulfonyl)aminocarbonyl]-4-[(aminocarbonyloxyethoxycarbonyl)-oxymethyl]-3-[((phenylmethoxy)carbonyl)amino]-2-azetidinone (C-3): $R_{30}$=$CH_2CH_2OC(=O)NH_2$; and

cis-(±)-1-[(Chlorosulfonyl)aminocarbonyl]-4-[(chloroethoxycarbonyl)oxymethyl]-3-[((phenylmethoxy)carbonyl)amino]-2-azetidinone (C-3): $R_{30}$=$CH_2CH_2Cl$.

Preparation 7 cis-(±)-1-[(5-Hydroxy-4-pyridone-2-yl)carbonylamino-1-(2-imidazolidone-3-yl)sulfonylaminocarbonyl]-4-[(methoxycarbonyl)oxymethyl]-3-[((phenylmethoxy)carbonyl)amino]-2-azetidinone (C-4: $R_{31}$=$CH_3$). Refer to Chart C.

N-Methyl-N-(trimethylsilyl)trifluoroacetamide, (1.35 ml) is added to a stirred suspension of 476 mg 1-(5-hydroxy-4-pyridone-2-yl)carbonylamino-2-imidazolidinone in 20 ml of ethyl acetate. Solution is complete in about 50 min. To this solution is added the title product (C-3) of Preparation 6 (prepared from 2 mmol of the title product (C-2) of Preparation 5 and dissolved in 5 ml of ethyl acetate). After 2.5 hours at ambient temperature, 0.4 ml of triethylamine is added, followed by water (10 ml) with good shaking. The aqueous layer is separated and acidified. The solids which precipitated are collected by filtration and dried. 805 mg of the title product (C-4) is obtained.

Physical characteristics are as follows:

$^{13}C$ NMR (δ, pyridine-$d_6$) 43.4, 45.5, 55.8, 59.2, 64.9, 67.2, 111.6, 128.3, 129.0, 137.2, 154.9, 157.4, 164.8.

FAB exact mass for [M·+H]+ found: 652.1333.

According to the procedures of Preparation 7, the following compounds are also prepared:

cis-(±)-1-[(5-Hydroxy-4-pyridone-2-yl)carbonylamino-1-(2-imidazolidone-3-yl)sulfonylaminocarbonyl]-4-[(formylaminoethoxycarbonyl)-oxymethyl]-3-[((phenylmethoxy)carbonyl)amino]-2-azetidinone (C-4): $R_{30}$=$CH_2CH_2NC(=O)H$;

cis-(±)-1-[(5-Hydroxy-4-pyridone-2-yl)carbonylamino-1-(2-imidazolidone-3-yl)sulfonylaminocarbonyl]-4-[(t-butoxycarbonylaminoethoxycarbonyl)oxymethyl]-3-[((phenylmethoxy)carbonyl)amino]-2-azetidinone (C-4): $R_{30}$=$CH_2CH_2NHBOC(t)$;

cis-(±)-1-[(5-Hydroxy-4-pyridone-2-yl)carbonylamino-1-(2-imidazolidone-3-yl)sulfonylaminocarbonyl]-4-[(aminocarbonyloxyethoxycarbonyl)oxymethyl]-3-[((phenylmethoxy)carbonyl)amino]-2-azetidinone (C-4): $R_{30}$=$CH_2CH_2OC(=O)NH_2$; and

cis-(±)-1-[(5-Hydroxy-4-pyridone-2-yl)carbonylamino-1-(2-imidazol-

idone-3-yl)sulfonylaminocarbonyl]-4-[(chloroethoxycarbonyl)oxymethyl]-3-[((phenylmethoxy)carbonyl)amino]-2-azetidinone (C-4): $R_{30}$=$CH_2CH_2Cl$.

Preparation 8 cis-(±)-3-Amino-1-[(5-hydroxy-4-pyridon-2-yl)carbonylamino-1-(2-imidazolidone-3-yl)sulfonylaminocarbonyl]-4-[(methoxycarbonyl)-oxymethyl]-2-azetidinone, N-potassium salt (C-5: $R_{31}$=$CH_3$). Refer to Chart C.

A solution of 200 mg of the N-potassium salt of the title product (C-4) of Preparation 7 (prepared by suspending the compound (C-4) in water and adjusting the pH to 6.0 with aqueous potassium bicarbonate, and lyophilizing to yield the N-potassium salt) in 5 ml of dimethylformamide containing 175 mg of palladium black is stirred under an atmosphere of hydrogen for 2 hours. The catalyst is removed by filtration. The filtrate is concentrated under vacuum. The residue is triturated with methanol and filtered. 123 mg of the title product (C-5) is obtained.

Physical characteristics are as follows:

$^{13}C$ NMR (δ, $Me_2CO$-$d_6$) 42.2, 44.0, 52.70, 55.2, 57.5, 58.0, 112.0, 126.1, 135.5, 147.9, 154.4, 156.0, 162.2, 164.5, 166.1, 167.8.

FAB exact mass found: 556.0497.

Example 1 cis-(±)-3-[2-(2-Amino-4-thiazolyl)-2-(1-carboxymethoxy)-imino]acetamido-4-N-formylglycinoyloxymethyl-1-[(5-hydroxy-4-pyridone-2-yl)carbonylamino-1-(2-imidazolidinone-3-yl)sulfonylaminocarbonylazetidine-2-one (B-7: n=1, $R_{40}$=1-carboxymethyl, $R_4$=-C(=O)H). Refer to Chart B.

(i) To a solution of the title product (B-5) of Preparation 3 (997 mg) and 2,6-lutidine (250 μl) in methylene dichloride (10.0 ml) at 0°C chlorosulfonyl isocyanate (190 μl) is added with stirring. This reaction mixture is stirred at 0° C for 2 hours to produce compound (B-6: $R_{40}$=1-t-butoxycarbonylmethyl, $R_4$=-C(=O)H).

(ii) To a solution of 1-(5-hydroxy-4-pyridone-2-yl)carbonylamino-2-imidazolidinone of Formula IV (525 mg) in acetonitrile (7.5 ml) at ambient temperature, N-methyl-N-(trimethylsilyl)-trifluoroacetamide (1.5 ml) is added and stirred for 2 hours.

(iii) The silylated imidazolidinone prepared in (ii) above is transferred to a solution of the compound (B-6) obtained in (i) above at 0°C and the reaction mixture is slowly warmed to ambient temperature

over 1 hour. The reaction mixture is stirred at ambient temperature for 3 hours. To the reaction mixture ethyl acetate (50 ml) and water (30 ml) are added and the organic layer is taken. The organic layer is dried over anhydrous sodium sulfate and concentrated under reduced pressure. The residual solid material is dissolved in trifluoroacetic acid (25 ml) at 0°C and it is warmed slowly to ambient temperature, at which temperature it is allowed to stay for 45 minutes. The reaction mixture is concentrated under reduced pressure and then it is dissolved in water by adjusting the pH of the solution to 4 to 4.5 by adding aqueous dibasic potassium phosphate solution. This solution is concentrated under reduced pressure and the residue material is passed through a column of Amberlite XAD-4 to obtain the title product (B-7).

Example 2 cis-(±)-3-[2-(2-Amino-4-thiazolyl)-(Z)-2-(methoxyimino)-acetamido]-1-[(5-hydroxy-4-pyridone-2-yl)carbonylamino-1-(2-imidazolidone-3-yl)sulfonylaminocarbonyl]-4-[(methoxycarbonyl)oxymethyl]-2-azetidinone, N-potassium salt (C-6:$R_{30}$=$CH_3$, $R_{40}$=$CH_3$). Refer to Chart C.

The N-potassium salt of the title product (C-4) of Preparation 7 (310 mg) is dissolved in 10 ml of dimethylformamide and 150 mg of palladium black is added. The mixture is stirred under an atmosphere of hydrogen for 2 hours. The catalyst is removed by filtration to give a solution of the title product (C-5) of Preparation 8 in dimethylformamide. To this solution is added 100 mg of 2-amino-4-thiazolyl-(Z)-2-(methoxyimino) acetic acid, 68 mg of 1-hydroxybenzotriazole hydrate and 103 mg of dicyclohexylcarbodiimide. The reaction mixture is stirred overnight. The solvent is evaporated under vacuum. The residue is stirred with 15 ml of acetone and filtered. The filtrate is diluted with water and the pH adjusted to 6 with potassium bicarbonate. The acetone is removed by distillation and the residue lyophilized to afford the crude product. This material is purified by dissolving in water and passing through a column of HP-20 resin. The column is washed with water and then water containing increasing amounts of acetonitrile. Fractions showing similar zones of inhibition when paper discs are dipped in them and spotted on an agar tray seeded with K. pneumoniae are combined and lyophilized to afford the title product (potassium salt of compound C-6), which shows FAB-MS [M·+H]+ at m/z 701.

Example 3 cis-(±)-3-[2-(2-Amino-4-thiazolyl)-(Z)-2-(carboxy-

methoxyimino)acetamido]-1-[(5-hydroxy-4-pyridon-2-yl)carbonylamino-1-(2-imidazolidone-3-yl)sulfonyl-aminocarbonyl]-4-[(methoxycarbonyl)oxymethyl]-2-azetidinone, (C-6):$R_{30}$=$CH_3$, $R_{40}$=$CH_2COOH$), dipotassium salt. Refer to Chart C.

1.2 g of the N-potassium salt of the title product (C-4) of Preparation 7 is dissolved in 20 ml of dimethylformamide containing 555 mg of palladium black and stirred under an atmosphere of hydrogen for 5 hours. The catalyst is removed by filtration. The title product (C-5) of Preparation 8 is not isolated. To the amine solution in dimethylformamide is added 739 mg of 2-(2-t-butoxycarbonylaminothiazole-4-yl)-(Z)-2-(t-butoxycarbonyl)methoxyiminoacetic acid, 249 mg of 1-hydroxybenzotriazole hydrate and 380 mg of dicyclohexylcarbodiimide. The reaction mixture is stirred at ambient temperature for 66 hours. The solution is filtered to remove the precipitate of dicyclohexylurea. The dimethylformamide is evaporated. The residue is dissolved in acetone and filtered to remove additional dicyclohexyl urea. The acetone is evaporated. The presence of the protected form of compound (C-6) with t-butoxycarbonyl on the amino thiazole $NH_2$- nitrogen, $R_{30}$=$CH_3$, $R_{40}$=$CH_2COOC(CH_3)_3$, N-potassium salt is identified by FAB mass spectral data. Observed (M·+1) m/z 901; (M·+Na) m/z 923; (M·+K)=939. A portion of crude product (500 mg) is dissolved in 15 ml of acetonitrile and 15 ml of water. This solution is passed through a column of 110 ml of HP-20 resin. The column is eluted with 300 ml of water and then with water-acetonitrile solutions containing an increasing percentage of acetonitrile. The fraction eluted with almost 100% acetonitrile is evaporated to yield 101 mg of residue. This material is dissolved in 1 ml of methylene dichloride and 0.5 ml of trifluoroacetic acid added. After 40 min this solution is added to 40 ml of 1:1 Skellysolve B-ether. The precipitate is collected by filtration and dried. This solid is dissolved in 4 ml of water at pH 6.5. The aqueous solution is passed through 13 ml of HP-20 resin. The column is washed with 80 ml of water followed by 60 ml of 10% acetonitrile-water. Fractions are combined on the basis of antibacterial activity vs. Pseudomonas aeruginosa and lyophilized. Dipotassium salt (C-6), the title product, is obtained as a white solid.

Physical characteristics are as follows:

FAB mass spectrum data: Observed [M·+H] m/z 745, [M·+Na] m/z 767,

[M·+K] m/z 783.

According to the procedures of Examples 3 and 4, the following compounds are also prepared:

cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(formylaminoethoxycarbonyl)-oxymethyl]-2-azetidinone, N-potassium salt (C-6): $R_{40}$=$CH_2COOH$, $R_{30}$=$CH_2$-$CH_2NHC(=O)H$);

cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(aminoethoxycarbonyl)-oxymethyl]-2-azetidinone, N-potassium salt (C-6): $R_{40}$=$CH_2COOH$, $R_{30}$=$CH_2CH_2$-$NH_2$);

cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(aminocarbonyloxyethoxycarbonyl)oxymethyl]-2-azetidinone, N-potassium salt (C-6): $R_{40}$=$CH_2COOH$, $R_{30}$=$CH_2CH_2OC(=O)NH_2$); and

cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(chloroethoxycarbonyl)-oxymethyl]-2-azetidinone, N-potassium salt (C-6): $R_{40}$=$CH_2COOH$, $R_{30}$=$CH_2$-$CH_2Cl$).

FORMULAS

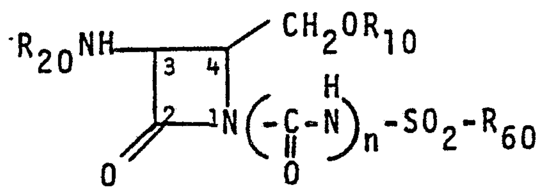

I

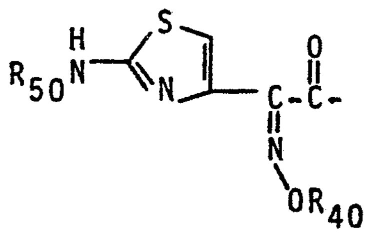

II

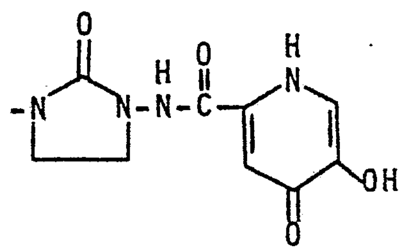

III

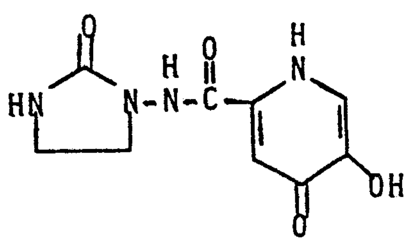

IV

CHART A

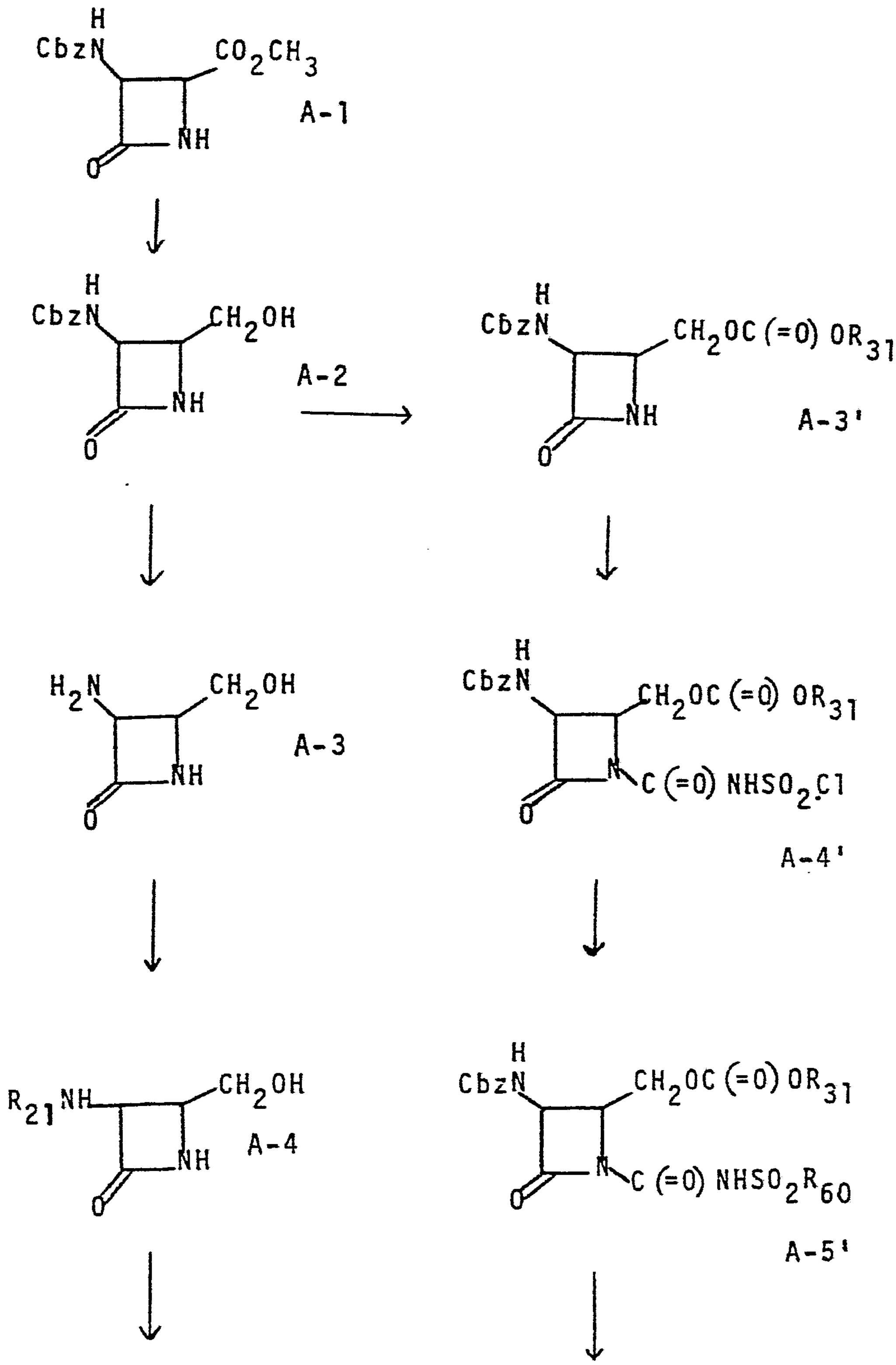

A-1

A-2

A-3'

A-3

A-4'

A-4

A-5'

CHART A (Continued)

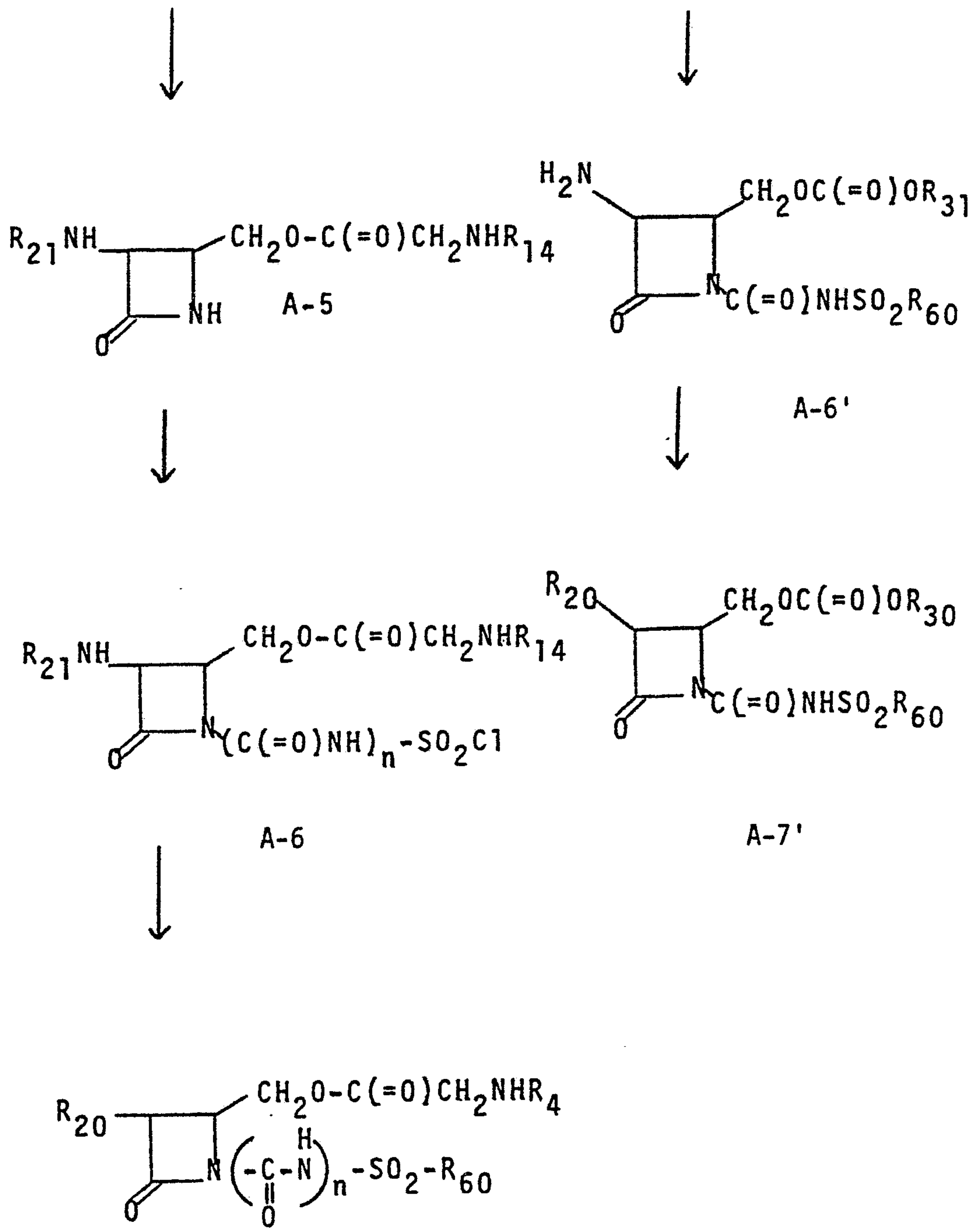

A-5

A-6'

A-6

A-7'

A-7

CHART B

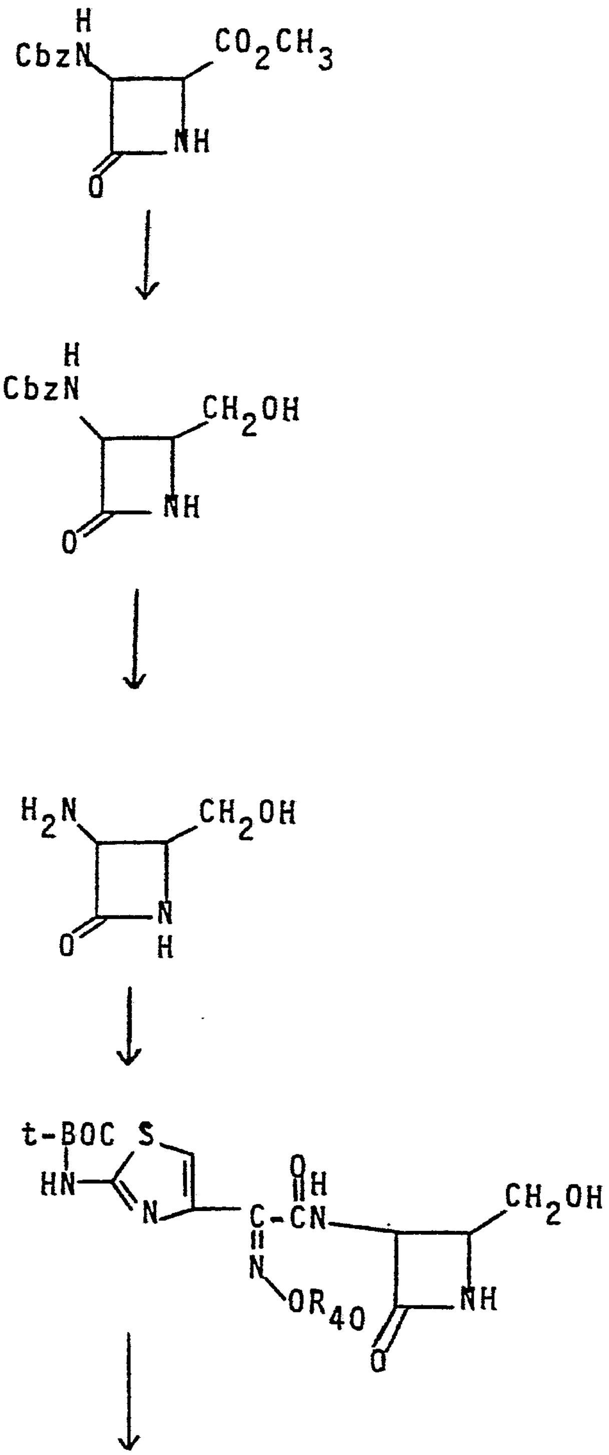

B-1

B-2

B-3

B-4

CHART B (Continued)

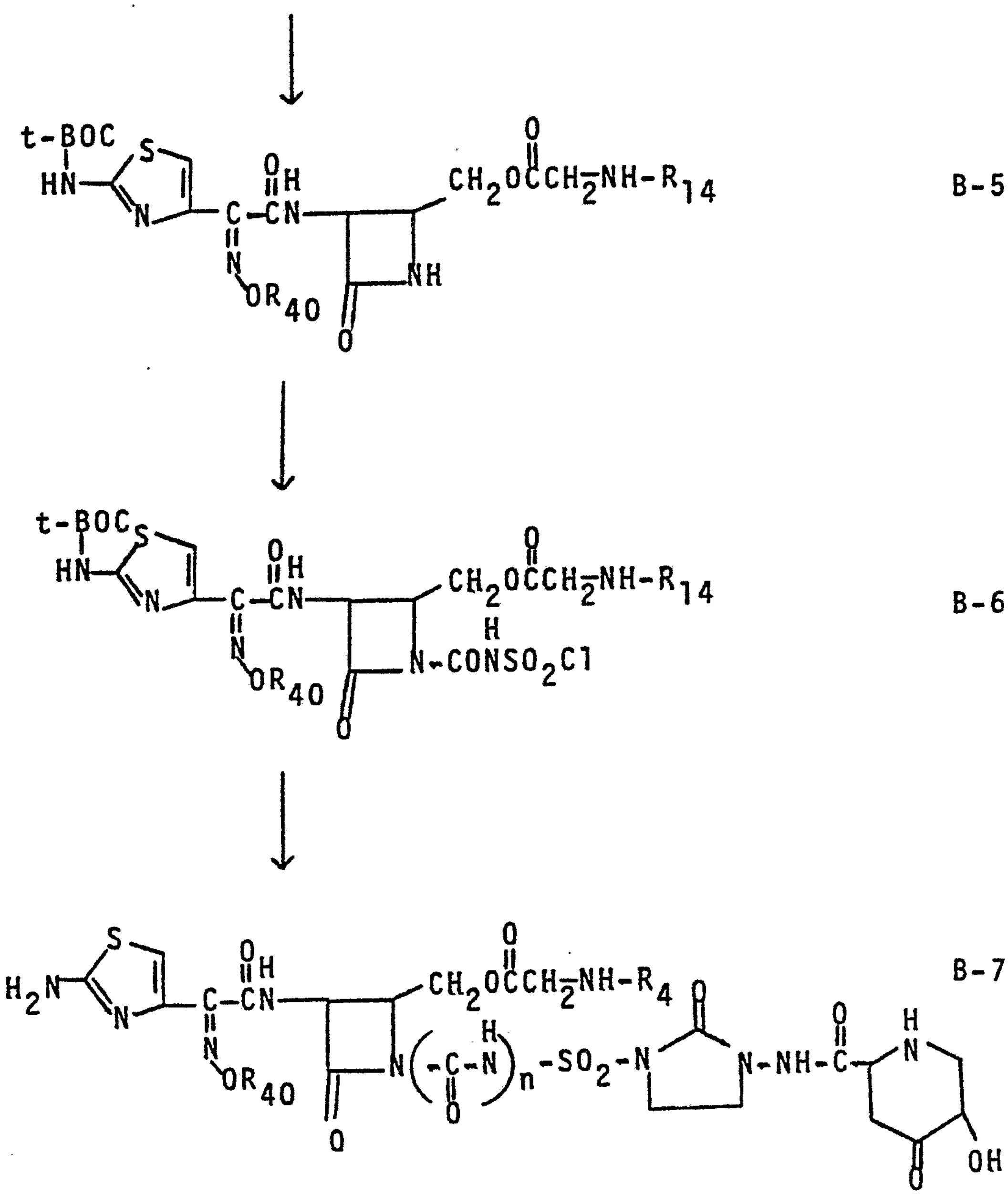

B-5

B-6

B-7

CHART C

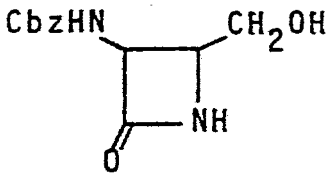

C-1 (B-2)

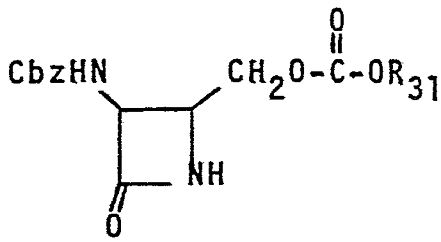

C-2

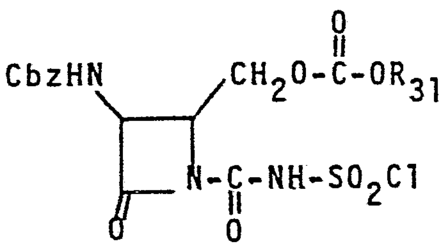

C-3

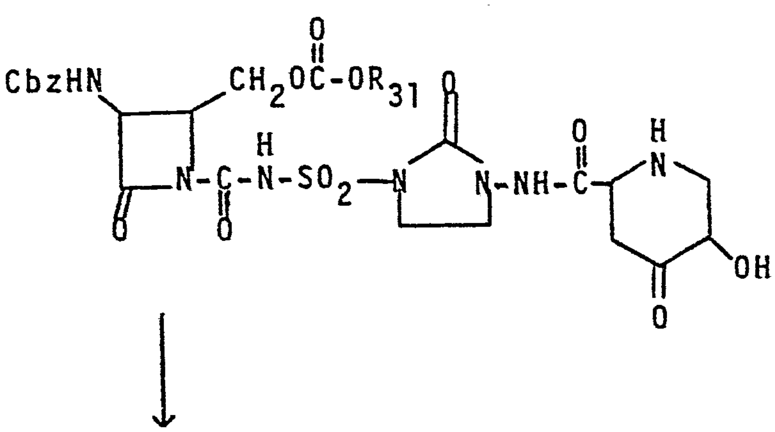

C-4

CHART C (Continued)

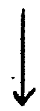

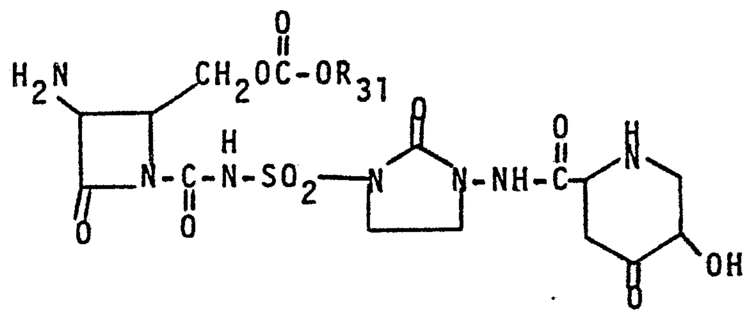

C-5

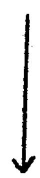

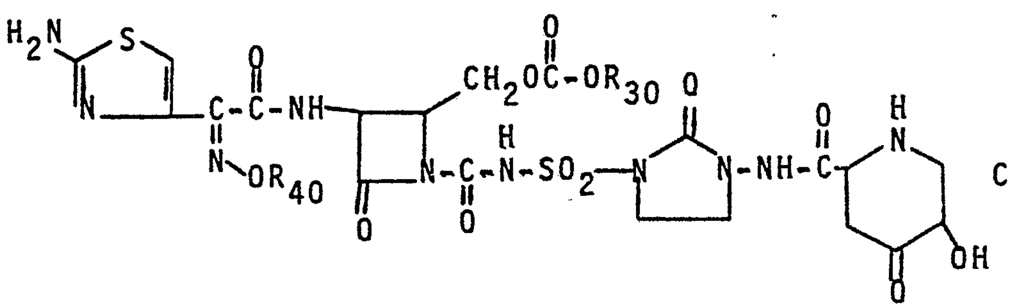

C-6

TABLE I

ANTIMICROBIAL IN VITRO TESTING

Minimum Inhibitory Concentration -MCG per ML-

| Organism Name | Culture No. | Compound A[1] | Compound B[2] |
|---|---|---|---|
| Ps. aeruginosa | 231 | 4 | 8 |
| | 9191 | 4 | 2 |
| | 6432 | 1 | 8 |
| E. coli | 9379 | ≤0.015 | 0.03 |
| | 9380 | ≤0.015 | 0.06 |
| | 9451 | ≤0.015 | 0.06 |
| K.pneumoniae | 58 | 1 | 0.03 |
| K. oxytoca | 9384 | 0.06 | 0.03 |

---

[1]Compound A is cis-(±)-3-[2-(2-Amino-4-thiazolyl)-(Z)-2-(carboxymethoxyimino)acetamido]-1-[(5-hydroxy-4-pyridon-2-yl)carbonylamino-1-(2-imidazolidone-3-yl)sulfonylaminocarbonyl]-4-[(methoxycarbonyl)-oxymethyl]-2-azetidinone, dipotassium salt.

[2]Compound B is 2-[[[1-(2-amino-4-thiazolyl)-2-[(2-methyl-4-oxo-1-sulfo-3-azetidinyl)amino]-2-oxoethylidene]amino]oxy]-2-methylpropanoic acid, also known as aztreonam.

CLAIMS

1. A compound of the formula I

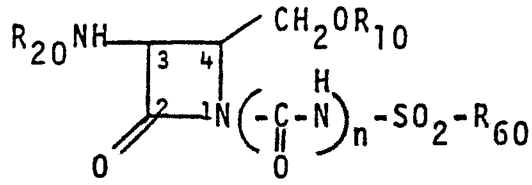

I

wherein the $R_{20}NH$- and $-CH_2OR_{10}$ groups are either cis or trans to each other;

wherein n is 1 or 2;

wherein $R_{10}$ is

a) $-C(=O)CH_2NHR_4$, or

b) $-C(=O)OR_{20}$;

provided that n is 2 only when $R_{10}$ is $-C(=O)CH_2NHR_4$;

wherein $R_4$ is

a) hydrogen,

b) $-C(=O)H$, or

c) t-butyloxycarbonyl;

wherein $R_{30}$ is

a) $-(C_1-C_8)$alkyl, or

b) $-(CH_2)_2X$ wherein X is $-NH_2$, -NH-(t-butyloxycarbonyl), $-NHCH_3$, $-N(CH_3)$-(t-butyloxycarbonyl), $-OC(=O)NH_2$, -Cl, $-OCH_3$ or $-NHC(=O)H$;

wherein $R_{20}$ is

a) hydrogen, or

b) a Z-oximino-acyl moiety of formula II

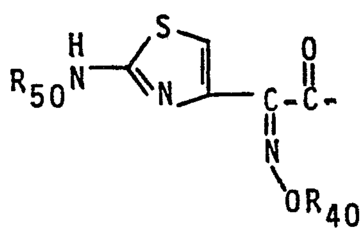

II

wherein $R_{40}$ is

a) $-CH_3$,

b) $-CH_2COOR_{41}$,

c) $-C(CH_3)_2COOR_{41}$, or

d) $-CH(CH_3)COOR_{41}$;

wherein $R_{41}$ is

a) hydrogen,

b) -t-butyl,

c) diphenylmethyl, or

d) benzyl;

wherein $R_{50}$ is

a) hydrogen,

b) t-butyloxycarbonyl,

c) benzyloxycarbonyl, or

d) triphenylmethyl;

wherein $R_{60}$ is an imidazolidinone moiety of formula III

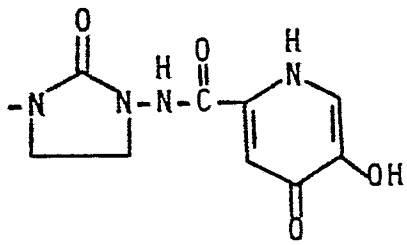

III

or a pharmaceutically acceptable salt thereof.

2. A compound of Claim 1

wherein n is 1 or 2;

wherein $R_{10}$ is $-C(=O)CH_2NHR_4$;

wherein $R_4$ is

a) hydrogen, or

b) $-C(=O)H$;

wherein $R_{20}$ is a Z-oximino-acyl moiety of formula II

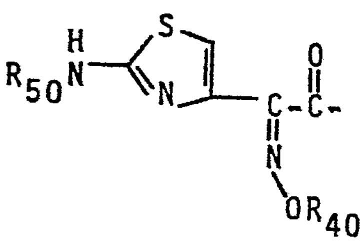

II

wherein $R_{40}$ is

a) $-CH_3$, or

b) $-CH_2COOR_{41}$;

wherein $R_{41}$ is hydrogen;

wherein $_{50}$ is hydrogen;

wherein $R_{60}$ is an imidazolidinone moiety of formula III

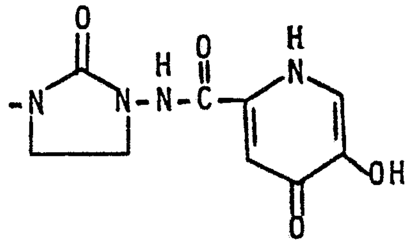

III

3. A compound of Claim 2 selected from the group consisting of:

a) cis-(±)-3-[2-(2-amino-4-thiazolyl)-2-methoxyiminoacetamido]-4-N-formylglycinoyl-oxymethyl-2-oxo-1-[(5-hydroxy-4-pyridone-2-yl)carbonylamino-1-(2-imidazolidinone-3-yl)sulfonylaminocarbonylazetidine-2-one;

b) cis-(±)-3-[2-(2-amino-4-thiazolyl)-2-(1-carboxymethoxy)-imino]acetamido]-4-N-formylglycinoyl-oxymethyl-1-[(5-hydroxy-4-pyridone-2-yl)carbonylamino-1-(2-imidazolidinone-3-yl)sulfonylaminocarbonylazetidine-2-one; and

c) cis-(±)-3-[2-(2-amino-4-thiazolyl)-2-(1-carboxymethoxy)-imino]acetamido]-4-N-glycinoyl-oxymethyl-1-[(5-hydroxy-4-pyridone-2-yl)carbonylamino-1-(2-imidazolidinone-3-yl)sulfonylaminocarbonyl-azetidine-2-one.

4. A compound of Claim 1
wherein n is 1;
wherein $R_{10}$ is $-C(=O)OR_{30}$;
wherein $R_{30}$ is

a) $-CH_3$, or
b) $-(CH_2)_2X$ wherein X is $-NH_2$, $-O(C=O)NH_2$, -Cl, or $-NHC(=O)H$;

wherein $R_{20}$ is a Z-oximino-acyl moiety of formula II

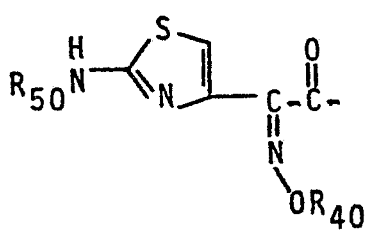

II

wherein $R_{40}$ is

a) $-CH_3$, or
b) $-CH_2COOR_{41}$;

wherein $R_{41}$ is hydrogen;
wherein $R_{50}$ is hydrogen;
wherein $R_{60}$ is an imidazolidinone moiety of formula III

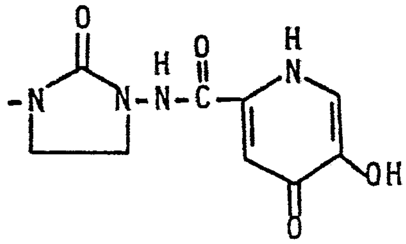

III

or a pharmaceutically acceptable salt thereof.

5. A compound of Claim 4 selected from the group consisting of:

a) cis-(±)-3-[2-(2-Amino-4-thiazolyl)-(Z)-2-(methoxyimino)acetamido]-1-[(5-hydroxy-4-pyridone-2-yl)carbonylamino-1-(2-imidazolidone-3-yl)-sulfonylaminocarbonyl]-4-[(methoxycarbonyl)oxymethyl]-2-azetidinone, N-potassium salt;

b) cis-(±)-3-[2-(2-Amino-4-thiazolyl)-(Z)-2-(carboxymethoxyimino)acetamido]-1-[(5-hydroxy-4-pyridon-2-yl)carbonylamino-1-(2-imidazolidone-3-yl)sulfonylaminocarbonyl]-4-[(methoxycarbonyl)oxymethyl]-2-azetidinone, dipotassium salt;

c) cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(formylaminoethoxycarbonyl)oxymethyl]-2-azetidinone, N-potassium salt;

d) cis-(±)-3-[2-Amino-4-thiazolyl)-(Z)-2-(carboxymethoxyimino)acetamido]-1-[(5-hydroxy-4-pyridone-2-yl)carbonylamino-1-(2-imidazolidone-3-yl)sulfonylaminocarbonyl]-4-[(aminoethoxycarbonyl)oxymethyl]-2-azetidinone, N-potassium salt;

e) cis-(±)-3-[2-Amino-4-thiazolyl)-(Z)-2-(carboxymethoxyimino)acetamido]-1-[(5-hydroxy-4-pyridone-2-yl)carbonylamino-1-(2-imidazolidone-3-yl)sulfonylaminocarbonyl]-4-[(aminocarbonyloxyethoxycarbonyl)oxymethyl]-2-azetidinone, N-potassium salt; and

f) cis-(±)-3-[2-(2-Amino-4-thiazolyl)-(Z)-2-(carboxymethoxyimino)-acetamido]-1-[(5-hydroxy-4-pyridone-2-yl)carbonylamino-1-(2-imidazolidone-3-yl)sulfonylaminocarbonyl]-4-[(chloroethoxycarbonyl)oxymethyl]-2-azetidinone, N-potassium salt.

0246786

European Patent Office

# EUROPEAN SEARCH REPORT

Application number

EP 87 30 4115

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | EP-A-0 085 291 (SQUIBB)<br>* Claims * | 1-5 | C 07 D 417/14<br>C 07 D 401/14 //<br>C 07 D 205/08<br>A 61 K 31/64 |
| Y | EP-A-0 062 876 (SQUIBB)<br>* Claims * | 1-5 | |
| P,Y | GB-A-2 181 130 (SQUIBB)<br>* Claims * | 1-5 | |

TECHNICAL FIELDS SEARCHED (Int Cl 4)

C 07 D 417/00
C 07 D 401/00
C 07 D 205/00
A 61 K 31/00

The present search report has been drawn up for all claims

| Place of search | Date of completion of the search | Examiner |
|---|---|---|
| THE HAGUE | 14-08-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82